# EUROPEAN PATENT APPLICATION

(11) **EP 3 653 107 A1**
(43) Date of publication of application: **20.05.2020**
(21) Application number: 18832464.4
(22) Date of filing: 08.06.2018
(51) Int. Cl.: A61B 3/117

(54) **IMAGE PROCESSING DEVICE, OPHTHALMIC OBSERVATION APPARATUS, AND OPHTHALMIC OBSERVATION SYSTEM**

(30) Priority: 12.07.2017 JP 2017136607
(71) Applicant: Sony Corporation, Tokyo 108-0075 (JP)
(72) Inventor: ENOKI, Junichiro, Tokyo 108-0075 (JP); SAKAGUCHI, Tatsumi, Tokyo 108-0075 (JP); OOTSUKI, Tomoyuki, Tokyo 108-0075 (JP); SOMA, Yoshio, Tokyo 108-0075 (JP)
(74) Representative: D Young & Co LLP
(86) International application number: PCT/JP2018/022052
(87) International publication number: WO 2019/012881

(57) **Abstract**

[Object] To provide an image processing apparatus, an ophthalmic observation apparatus, and an ophthalmic observation system that are suitable for observation of eyes.

[Solving Means] An image processing apparatus according to the present technology includes an image generation unit. The image generation unit inverts, in a captured image captured via a front lens attached to an eye, an inverted area that is an image area in which an image is inverted by the front lens in the image area, and generates a display image.

## Description

### Technical Field

The present technology relates to an image processing apparatus, an ophthalmic observation apparatus, and an ophthalmic observation system that are used for ophthalmic surgery.

### Background Art

Observation of an eyeball from the front side is widely performed in ophthalmic diagnosis and surgical treatment. Particularly in glaucoma disease, observation of the anterior chamber angle at the root of the iris and the cornea has been regarded as important also in diagnosis.

In recent years, it has been practiced to perform an operation while directly observing this corner angle during treatment even in extremely minimally invasive glaucoma surgery called MIGS (minimally invasive glaucoma surgery). However, light from the anterior chamber angle is totally reflected by the cornea, which makes it difficult to perform observation as it is.

For this reason, it is common to perform observation by combining a contact lens called a gonio lens with a surgical microscope or a slit lamp. However, since most gonio lenses have a narrow field of view, it is necessary to properly position both the gonio lens and the microscope in accordance with an object to be observed in order to see the position to be observed. In order to cope with this problem, Patent Literature 1 proposes a gonio lens capable of checking the entire circumference of the corner angle using a concave lens.

### Citation List

### Patent Literature

Patent Literature 1: Japanese Unexamined Patent Application Publication No. 2000-504251

### Disclosure of Invention

### Technical Problem

However, in the gonio lens described in Patent Literature1, the use of a concave lens reverses the surgical field, which confuses an operator. In order to prevent his, it is necessary to further combine a plurality of lenses or mirrors, and thus loss such as attenuation tends to occur.

Further, since an image formed using a lens is observed, in the case of magnifying an arbitrary position, it takes trouble to properly operate an optical microscope. In particular, in the case where the surgical field has been reversed, confusion tends to occur because the operation direction and the surgical field are inconsistent.

In view of the circumstances as described above, it is an object of the present technology to provide an image processing apparatus, an ophthalmic observation apparatus, and an ophthalmic observation system that are suitable for observation of eyes.

### Solution to Problem

In order to achieve the above-mentioned object, an image processing apparatus according to an embodiment of the present technology includes an image generation unit.

The image generation unit inverts an inverted area in a captured image captured via a front lens attached to an eye, the inverted area being an image area in which an image is inverted by the front lens, and generates a display image.

With this configuration, since the inversion of the image due to the front lens is eliminated by image processing, it is possible to prevent a surgeon from being confused due to use of the front lens.

The image processing apparatus may further include an area detection unit that detects the inverted area in the captured image.

The image generation unit may invert the inverted area with a center of the inverted area as a center point.

The image generation unit may invert the inverted area and synthesize the area with a non-inverted area to generate the display image, the non-inverted area being an image area in which an image is not inverted by the front lens in the captured image.

The front lens may be a direct-view front lens that includes a concave lens and a convex lens, the concave lens being in contact with a cornea, the convex lens refracting light emitted from the concave lens toward a front direction of the eye, and the inverted area may be circular and the non-inverted area may surround a periphery of the inverted area.

The image generation unit may invert the inverted area, and synthesize the inverted area with the non-inverted area by causing an outer periphery of the inverted area, which has been inverted, and an inner periphery of the non-inverted area to match with each other.

The front lens may be a reflection front lens that includes a convex lens and a mirror, the convex lens being in contact with a cornea, the mirror being circumferentially disposed around the convex lens and reflecting light emitted from the convex lens toward a front direction of the eye, and the inverted area may be a circumferential area and the non-inverted area may include a center area and an outer peripheral area, the center area being surrounded by the inverted area, the outer peripheral area surrounding the inverted area.

The image generation unit may detect, as an unnecessary area, an area including an image of the center area in the inverted area, delete, the unnecessary area, invert the inverted area, and synthesize the inverted area, the center area, and the outer peripheral area with each other by causing an inner periphery of the inverted area, which has been inverted, and an outer periphery of the center area to match with each other and causing an outer periphery of the inverted area, which has been inverted, and an inner periphery of the center area to match with each other.

The image processing apparatus may further include a mode determination unit that detects that the captured image incudes the front lens, selects a correction mode for correcting inversion of an image by the front lens, and notifies the area detection unit and the image generation unit of the correction mode, in which the area detection unit may detect, where the mode determination unit has selected the correction mode, the inverted area.

The mode determination unit may detect the front lens by object recognition processing on the captured image.

The mode determination unit may detect the front lens by detecting a marker attached to the front lens in the captured image.

The area detection unit may detect the inverted area by object recognition processing on the captured image.

The area detection unit may detect the inverted area by using a difference in texture due to a structure of the eye in the captured image.

The area detection unit may detect the inverted area by edge detection processing on the captured image.

The area detection unit may detect the inverted area on a basis of a difference between the captured image and a captured image of the eye to which the front lens is not attached.

The area detection unit may detect the inverted area by using depth information extracted from parallax information obtained from the captured image.

The captured image may include a plurality of images captured for each predetermined imaging range, and the image generation unit may invert a position and an orientation of each of the plurality of images to generate the display image.

In order to achieve the above-mentioned object, an ophthalmic observation apparatus according to an embodiment of the present technology includes: a front lens; and an image processing apparatus.

The front lens is attached to an eye and inverts an image.

The image processing apparatus includes an image generation unit that inverts an inverted area in a captured image captured via the front lens, the inverted area being an image area in which an image is inverted by the front lens, and generates a display image.

The front lens may be a gonio lens.

In order to achieve the above-mentioned object, an ophthalmic observation system according to an embodiment of the present technology includes: a front lens; a microscope; a microscope control input unit; an imaging apparatus; an image processing apparatus; and a microscope control unit.

The front lens is attached to an eye and inverts an image.

The microscope magnifies emitted light of the front lens.

The microscope control input unit accepts an operation input by a user and generates an input signal.

The imaging apparatus is connected to the microscope and captures an image via the front lens and the microscope.

The image processing apparatus includes an image generation unit that inverts an inverted area in the captured image captured via the front lens, the inverted area being an image area in which an image is inverted by the front lens, and generates a display image.

The microscope control unit inverts, where the image generation unit has inverted the inverted area, the input signal to control the microscope.

### Advantageous Effects of Invention

As described above, in accordance with the present technology, it is possible to provide an image processing apparatus, an ophthalmic observation apparatus, and an ophthalmic observation system that are suitable for observation of eyes. It should be noted that the effect described here is not necessarily limitative and may be any effect described in the present disclosure.

### Brief Description of Drawings

[Fig. 1] Fig. 1 is a block diagram showing a configuration of an ophthalmic observation system according to a first embodiment of the present technology.
[Fig. 2] Fig. 2 is a schematic diagram showing a structure of an eye that is an object to be observed by the observation system.
[Fig. 3] Fig. 3 is a schematic diagram showing a structure of the eye that is the object to be observed by the observation system.
[Fig. 4] Fig. 4 is a schematic diagram showing a direct-view front lens of the observation system.
[Fig. 5] Fig. 5 is a schematic diagram showing a reflection front lens of the observation system.
[Fig. 6] Fig. 6 is a schematic diagram showing an optical path in the direct-view front lens of the observation system.
[Fig. 7] Fig. 7 is a schematic diagram showing an image of an eye, which is formed by the direct-view front lens of the observation system.
[Fig. 8] Fig. 8 is a schematic diagram showing an inverted area the image of the eye, which is formed by the direct-view front lens of the observation system.
[Fig. 9] Fig. 9 is a schematic diagram showing arrangement of mirrors of the reflection front lens of the observation system.
[Fig. 10]Fig. 10 is a schematic diagram showing an optical path in the reflection front lens of the observation system.
[Fig. 11]Fig. 11 is a schematic diagram showing an image of the eye, which is formed by the reflection front lens of the observation system.
[Fig. 12] Fig. 12 is a schematic diagram showing an inverted area of the image of the eye, which is formed by the reflection front lens of the observation system.
[Fig. 13]Fig. 13 is a schematic diagram showing the positional relationship between the image of the eye, which is formed by the reflection front lens of the observation system, and an eye part.
[Fig. 14]Fig. 14 is a schematic diagram showing a display image generated by an image processing apparatus of the observation system.
[Fig. 15]Fig. 15 is a block diagram showing a functional configuration of the image processing apparatus of the observation system.
[Fig. 16]Fig. 16 is a schematic diagram showing an inverted area detected by an area detection unit of the image processing apparatus of the observation system.
[Fig. 17]Fig. 17 is a schematic diagram showing generation of a display image by an image generation unit of the image processing apparatus of the observation system.
[Fig. 18]Fig. 18 is a schematic diagram showing an inverted area detected by the area detection unit of the image processing apparatus of the observation system.
[Fig. 19]Fig. 19 is a schematic diagram showing an unnecessary area detected by the area detection unit of the image processing apparatus of the observation system.
[Fig. 20]Fig. 20 is a schematic diagram showing generation of a display image by the image generation unit of the image processing apparatus of the observation system.
[Fig. 21]Fig. 21 is a schematic diagram showing generation of a display image by the image generation unit of the image processing apparatus of the observation system.
[Fig. 22]Fig. 22 is a flowchart showing an operation of the image processing apparatus of the observation system.
[Fig. 23]Fig. 23 is a block diagram showing a hardware configuration of the image processing apparatus of the observation system.
[Fig. 24]Fig. 24 is a block diagram showing a configuration of an ophthalmic observation system according to a second embodiment of the present technology.
[Fig. 25]Fig. 25 is a schematic diagram showing a configuration of a front lens and an imaging apparatus of the observation system.
[Fig. 26]Fig. 26 is a schematic diagram showing a configuration of the front lens and the imaging apparatus of the observation system.
[Fig. 27]Fig. 27 is a schematic diagram showing a configuration of the front lens and the imaging apparatus of the observation system.
[Fig. 28]Fig. 28 is a schematic diagram showing arrangement of small cameras of an imaging apparatus of the observation system.
[Fig. 29]Fig. 29 is a schematic diagram showing an imaging range of a captured image captured by the imaging apparatus of the observation system.
[Fig. 30]Fig. 30 is a block diagram showing a functional configuration of the image processing apparatus of the observation system.
[Fig. 31]Fig. 31 is a schematic diagram showing generation of a display image by the image processing apparatus of the observation system.
[Fig. 32]Fig. 32 is a schematic diagram showing a display image generated by the image processing apparatus of the observation system.
[Fig. 33]Fig. 33 is a flowchart showing an operation of the image processing apparatus of the observation system.

### Mode(s) for Carrying Out the Invention

### (First Embodiment)

An ophthalmic observation system according to a first embodiment of the present technology will be described.

### [Configuration of Ophthalmic Observation System]

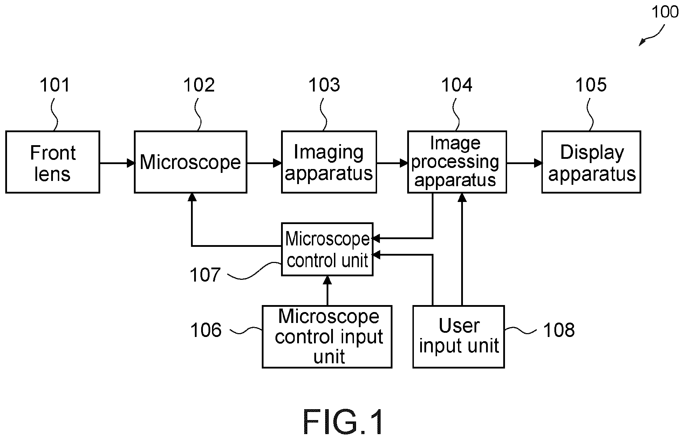

Fig. 1 is a block diagram showing a configuration of an ophthalmic observation system 100 according to this embodiment. As shown in the figure, the ophthalmic observation system 100 includes a front lens 101, a microscope 102, an imaging apparatus 103, an image processing apparatus 104, a display apparatus 105, a microscope control input unit 106, a microscope control unit 107, and a user input unit 108.

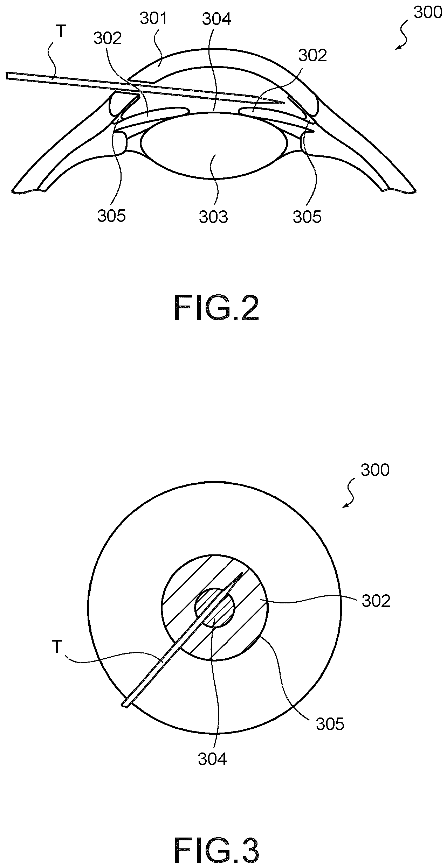

Fig. 2 and Fig. 3 are each a schematic diagram showing a structure of an eye 300 that is an object to be observed by the ophthalmic observation system 100. Fig. 2 is a cross-sectional view, and Fig. 3 is a plan view of the eye as viewed from the front direction. As shown in the figures, the eye 300 includes tissues such as a cornea 301, irises 302, and a crystalline lens 303. There is a pupil 304 between the irises 302 on the surface of the crystalline lens 303, and a corner angle 305 is located on the periphery of the cornea 301. Further, a surgical instrument T to be used for eye surgery is shown in Fig. 2 and Fig. 3.

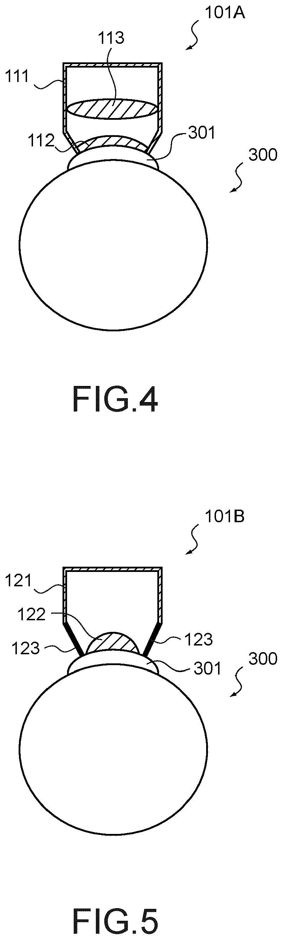

The front lens 101 is a lens to be attached to an eye. Fig. 4 and Fig. 5 are each a schematic diagram showing the front lens 101. The front lens 101 may be a direct-view front lens (hereinafter, direct-view front lens 101A) shown in Fig. 4, or a reflection front lens (hereinafter, reflection front lens 101B) shown in Fig. 5.

As shown in Fig. 4, the direct-view front lens 101A is placed on the cornea 301, and includes a support member 111, a concave lens 112, and a convex lens 113. The concave lens 112 is in contact with the cornea 301, and the convex lens 113 is provided away from the concave lens 112.

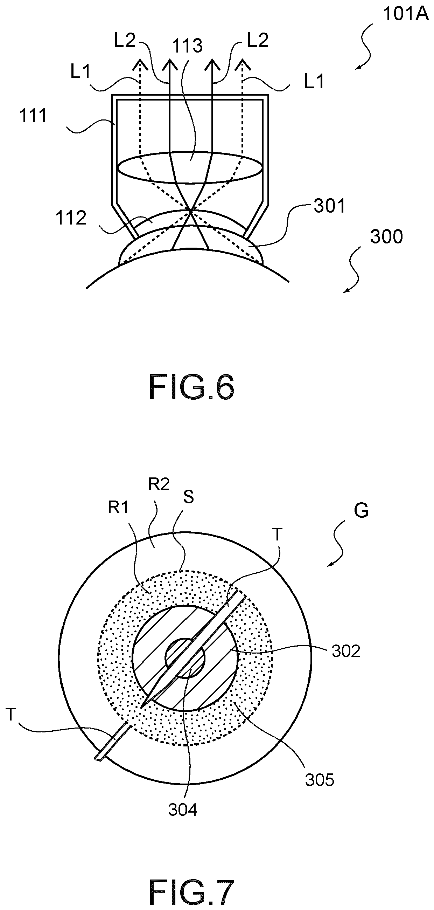

Fig. 6 is a schematic diagram showing an optical path in the direct-view front lens 101A. As shown in the figure, light (L1 in the figure) emitted from the periphery of the iris 302, such as the corner angle 305, is inverted by the concave lens 112 and the convex lens 113, and emitted toward the front direction of the eye. Further, also light (L2 in the figure) emitted from the center of the iris 302, such as the pupil 304, is inverted by the concave lens 112 and the convex lens 113, and output toward the front direction of the eye.

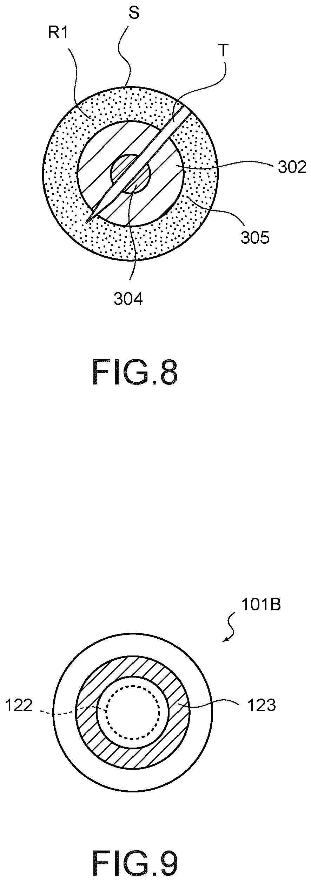

Fig. 7 is a schematic diagram showing an image of the eye viewed via the direct-view front lens 101A. As shown in the figure, an image of the corner angle 305 shown by the direct-view front lens 101A appears around the iris 302, and the image is inverted in the area inside a periphery S of the direct-view front lens 101A. Further, the image is magnified to a certain extent by the direct-view front lens 101A. In Fig. 7, the area in which the image is inverted is shown as an inverted area R1, and the area in which the image is not inverted is shown as a non-inverted area R2. Fig. 8 is a schematic diagram showing only the inverted area R1.

As shown in Fig. 5, the reflection front lens 101B is placed on the cornea 301, and includes a support member 121, a convex lens 122, and a mirror 123. The convex lens 122 is disposed to be in contact with the cornea 301. Fig. 9 is a schematic diagram showing arrangement of the mirror 123, and is a diagram as viewed from the front direction of the eye. As shown in the figure, the mirror 123 is disposed circumferentially around the convex lens 122.

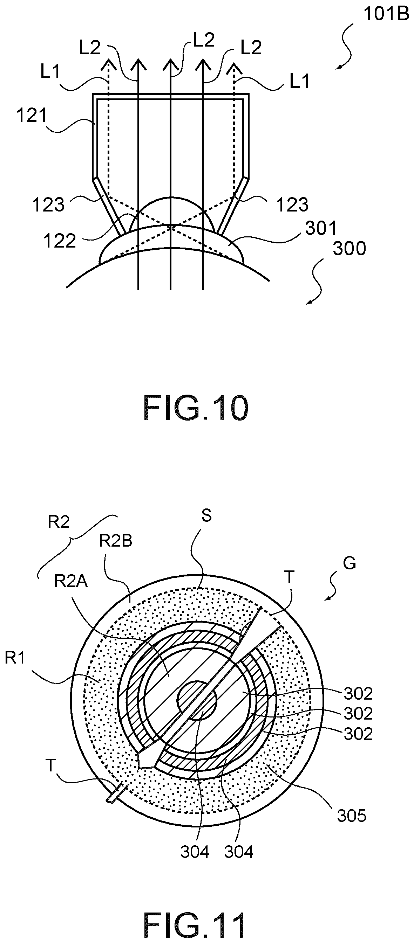

Fig. 10 is a schematic diagram showing an optical path in the reflection front lens 101B. As shown in the figure, light (L1 in the figure) emitted from the periphery of the iris 302, such as the corner angle 305, is refracted by the convex lens 122, reflected by the mirror 123 toward the front direction of the eye, and inverted and output. Further, light (L2 in the figure) emitted from the center of the iris 302, such as the pupil 304, is not reflected by the mirror 123 and is output without being inverted.

Fig. 11 is a schematic diagram showing an image of the eye viewed via the reflection front lens 101B. As shown in the figure, an image of the corner angle 305 shown by the reflection front lens 101B appears around the iris 302. Further, the image is inverted as indicated by the light L1 in the area within a certain range inside the periphery S of the reflection front lens 101B. Hereinafter, the area in which the image is inverted will be referred to as the inverted area R1, and the area in which the image is not inverted will be referred to as the non-inverted area R2.

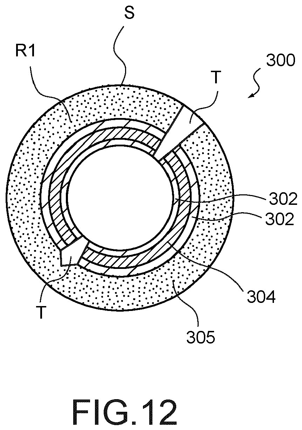

Fig. 12 is a schematic diagram showing only the inverted area R1. Since the mirror 123 is circumferentially disposed, also the inverted area R1 is a circumferential area as shown in the figure. Further, as indicated by the image of the surgical instrument T, the image is gradually magnified from the inner periphery side to the outer periphery side of the inverted area R1.

Further, as shown in Fig. 11, the non-inverted area R2 includes a center area R2A inside the inverted area R1 and an outer peripheral area R2B outside the inverted area R1. The center area R2A is an area in which light is not reflected by the mirror 123 as indicated by the light L2, and the peripheral area 2B is an area outside the reflection front lens 101B.

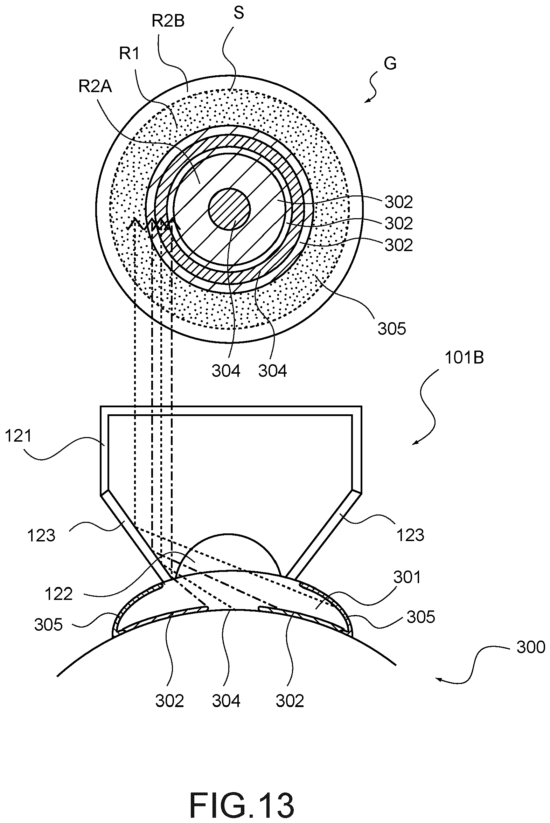

Fig. 13 is a schematic diagram showing the relationship between the structure of the eye and the image generated by the reflection front lens 101B. As shown in the figure, the inverted area R1 includes images of the corner angle 305, the iris 302, and the pupil 304 reflected by the mirror 123. For this reason, the images of the iris 302 and the pupil 304 appear in both the center area R2A and the inverted area R1.

The front lens 101 has the configuration as described above. The front lens 101 only needs to be a lens that inverts a part of the image, the positional relationship between the lens and the eye being directly or indirectly fixed. Typically, the front lens 101 is a gonio lens for observing the corner angle 305. Further, the front lens 101 may be a wide view lens used in a wide view system for retinal observation.

The microscope 102 magnifies the light emitted from the front lens 101, and causes the light to enter the imaging apparatus 103. The microscope 102 can be an optical microscope having a general configuration. Further, the microscope 102 may be one provided with two lens barrels for the right and left eyes. The microscope 102 is favorably capable of moving the field of view in the X-Y direction. The magnification, the field of view range, and the like of the microscope 102 are controlled by the microscope control unit 107.

The imaging apparatus 103 is mounted on the microscope 102, and captures an image of an eye via the front lens 101 and the microscope 102. The image captured by the imaging apparatus 103 (hereinafter, captured image) is an image as shown in Fig. 3 in the case where the front lens 101 is not attached to the eye, and an image as shown in Fig. 7 or Fig. 11 in the case where the front lens 101 is attached to the eye. The imaging apparatus 103 outputs the captured image to the image processing apparatus 104.

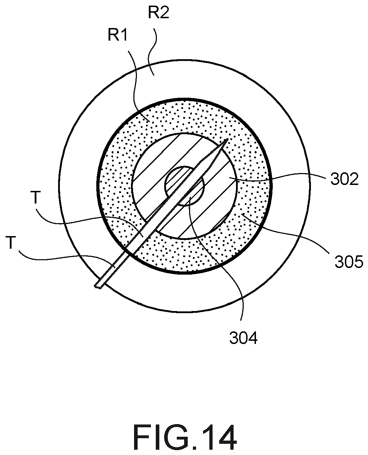

The image processing apparatus 104 performs image processing on the captured image output from the imaging apparatus 103 to generate a display image. Fig. 14 is a schematic diagram showing a display image. The image processing apparatus 104 inverts, in the case where the captured image includes the front lens 101, the inverted area R1 in the captured image to correct it in the correct orientation as shown in Fig. 14. Details of this will be described below. The image processing apparatus 104 outputs the generated display image to the display apparatus 105 for display. Further, the image processing apparatus 104 outputs, to the microscope control unit 107, information regarding inversion such as presence/absence of inversion and the range of the inverted area.

The display apparatus 105 displays the display image output from the image processing apparatus 104. The display apparatus 105 is a general display or head mounted display. Further, the display apparatus 105 may include a plurality of displays, e.g., a display for a surgeon and a display for an assistant.

The microscope control input unit 106 accepts an operation input to the microscope 102 by a user. The user is capable of performing an operation input of moving the field of view of the microscope 102 in the X-Y direction (hereinafter, X-Y operation) by using the microscope control input unit 106. The microscope control input unit 106 is, for example, a foot switch. The microscope control input unit 106 generates an input signal on the basis of a user operation, and supplies the input signal to the microscope control unit 107.

The microscope control unit 107 controls the microscope 102 on the basis of the input signal supplied from the microscope control input unit 106. The microscope control unit 107 is capable of adjusting the position of the lens barrel of the microscope 102, or the like in accordance with the input signal to move the field of view of the microscope 102.

Here, the microscope control unit 107 acquires information regarding inversion from the image processing apparatus 104, and inverts, in the case where inversion has been performed, the input signal supplied from the microscope control input unit 106 in the inverted area R1.

In the case where inversion of the inverted area R1 has been performed in the image processing apparatus 104 as described above, when the user moves the field of view, since the display image includes the inverted image of the eye, the field of view is moved in the opposite direction if the user tries to move the field of view in the direction that the user desires to see. This confuses the user.

Meanwhile, the microscope control unit 107 inverts the input signal of the X-Y operation, the field of view of the microscope 102 is moved in accordance with the X-Y operation intended by the user, which eliminates the confusion of the user.

The user input unit 108 sets, in accordance with a user instruction, whether or not to execute the above-mentioned processing of inverting the input signal by the microscope control unit 107.

### [Functional Configuration of Image Processing Apparatus]

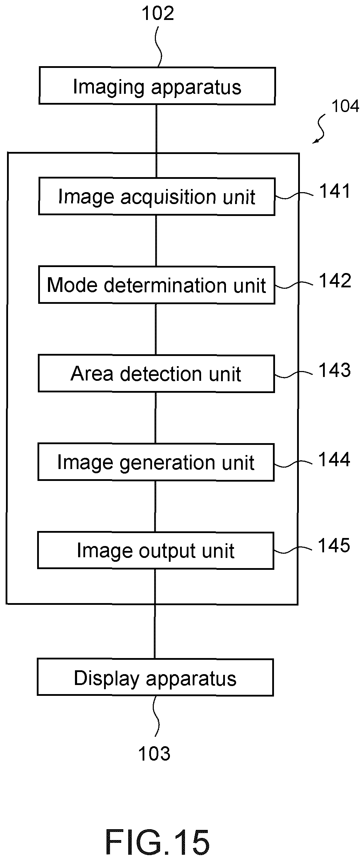

Fig. 15 is a block diagram showing a functional configuration of the image processing apparatus 104. As shown in the figure, the image processing apparatus 104 includes an image acquisition unit 141, a mode determination unit 142, an area detection unit 143, an image generation unit 144, and an image output unit 145.

### <1. Case of Direct-View front lens>

The functional configuration of the image processing apparatus 104 in the case where the direct-view front lens 101A (see Fig. 4) is used as the front lens 101 will be described.

The image acquisition unit 141 acquires a captured image from the imaging apparatus 103. The captured image in the case where the direct-view front lens 101A is used is an image as shown in Fig. 7. As described above, the captured image includes the inverted area R1 and the non-inverted area R2. The image acquisition unit 141 supplies the acquired captured image to the mode determination unit 142.

The mode determination unit 142 determines the operation mode of the image processing apparatus 104. Specifically, the mode determination unit 142 selects any of a mode (hereinafter, correction mode) for correcting the influence by the front lens 101 and a normal mode (hereinafter, normal mode).

The mode determination unit 142 is capable of determining the mode using a result of detecting the front lens 101 in the captured image.

The mode determination unit 142 is capable of detecting the front lens 101 by an object recognition technology that detects, in the captured image, features of the image of the front lens 101 stored in a database in advance. Further, the mode determination unit 142 may detect the front lens 101 by detecting a marker attached to the front lens 101.

The mode determination unit 142 selects the correction mode and the normal mode in the case where the captured image includes the front lens 101 and the case where the captured image does not include the front lens 101, respectively.

Further, the mode determination unit 142 may determine the mode in accordance with a user instruction input using the user input unit 108. The mode determination unit 142 notifies the area detection unit 143 and the image generation unit 144 of the determined mode.

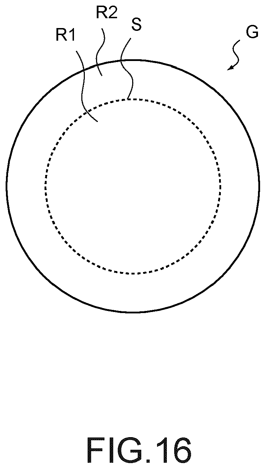

The area detection unit 143 detects the inverted area R1 in the captured image. Fig. 16 is a schematic diagram showing the range of the inverted area R1 in a captured image G captured in the case where the direct-view front lens 101A is attached to an eye.

The area detection unit 143 is capable of detecting the inverted area R1 by using the above-mentioned object recognition. Further, the area detection unit 143 is also capable of detecting the inverted area R1 using the fact that the outside of the front lens 101 is an area of a white eye while the inside of the front lens 101, such as the corner angle, the iris, and the pupil, has color and texture different from those of the white eye.

Further, since there is a clear edge in the periphery S of the front lens 101 in the captured image, the area detection unit 143 is also capable of performing edge detection processing and detecting the area inside the detected edge as the inverted area R1.

The above-mentioned detection method detects the inverted area R1 from one captured image. However, the area detection unit 143 is also capable of detecting the inverted area R1 by using a plurality of captured images. Specifically, the area detection unit 143 may hold a captured image at the start of surgery in which the front lens 101 is not attached, compare the captured image with a captured image including the front lens 101, and detects the area having a large difference as the inverted area R1.

Further, in the case where the imaging apparatus 103 is mounted on the microscope 102, it is possible to acquire both captured images for the left and right eyes. The area detection unit 143 is also capable of extracting depth information from information regarding the parallax between the two captured image, and detecting the inverted area R1 by using the fact that the front lens 101 is in front of the eye.

Note that in the case where the area detection unit 143 has successfully detected the inverted area R1 once, the area detection unit 143 may detect the inverted area R1 by tracking the detected front lens 101. The area detection unit 143 detects the inverted area R1 in this way. The area detection unit 143 supplies the range of the detected inverted area R1 to the image generation unit 144.

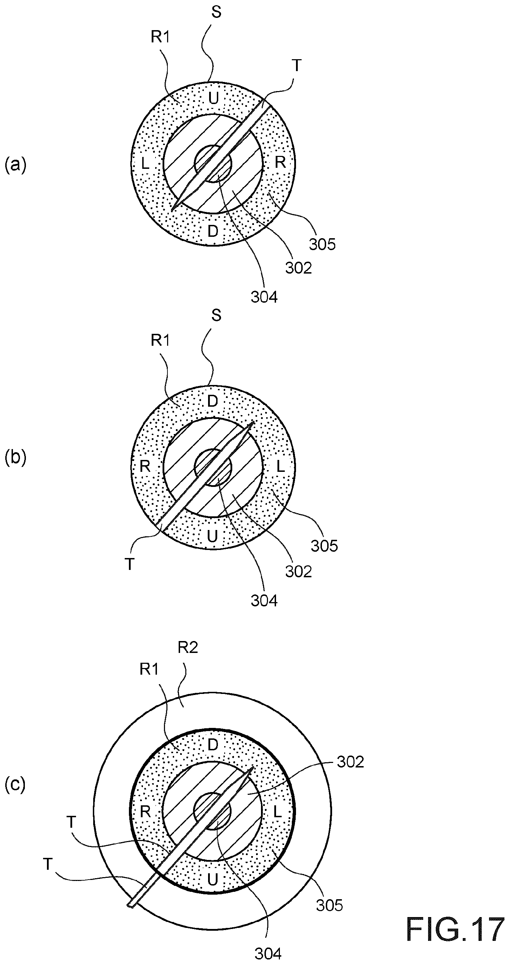

The image generation unit 144 performs correction processing on the captured image to generate a display image. Fig. 17 is a schematic diagram showing an aspect of the correction processing by the image generation unit 144. First, the image generation unit 144 extracts the inverted area R1 in the captured image G as shown in Part (a) of Fig. 17. In the figure, R, L, U, and D are each a symbol indicating the position in the inverted area R1.

Subsequently, the image generation unit 144 inverts the inverted area R1 so as to be point-symmetric with respect to the center of the inverted area R1 as shown in Part (b) of Fig. 17. Further, as shown in Part (C) of Fig. 17, the image generation unit 144 synthesizes the inverted area R1 with the non-inverted area R2 by causing the outer periphery of the inverted area R1, which has been inverted, and the inner periphery of the non-inverted area R2 to match with each other.

As a result, the image generation unit 144 is capable of generating a display image in which the inversion has been eliminated as shown in Fig. 14. The image generation unit 144 supplies the generated display image to the image output unit 145.

The image output unit 145 outputs the display image to the display apparatus 105 and causes the display apparatus 105 to display the display image. Here, in ophthalmic surgery, in the case where there are a surgeon and an assistant, the surgeon and the assistant are often in an orthogonal positional relationship. In this case, the image output unit 145 is capable of causing a display to display the display image viewed from the position of the surgeon and another display to display the display image rotated in accordance with the position of the assistant. As a result, it is possible to provide a display image that is not likely to confuse both the surgeon and the assistant.

### <2. Case of Reflection front lens>

The functional configuration of the image processing apparatus 104 in the case where the reflection front lens 101B (see Fig. 5) is used as the front lens 101 will be described.

The image acquisition unit 141 acquires a captured image from the imaging apparatus 103. The captured image in the case where the reflection front lens 101B is used is an image as shown in Fig. 11. As described above, the captured image includes the inverted area R1 and the non-inverted area R2. The image acquisition unit 141 supplies the acquired captured image to the mode determination unit 142.

The mode determination unit 142 determines the operation mode of the image processing apparatus 104. The mode determination unit 142 is capable of determining the operation mode in accordance with a result of detecting the front lens 101 in the captured image or a user instruction similarly to the case of the direct-view front lens 101A. The mode determination unit 142 notifies the area detection unit 143 and the image generation unit 144 of the determined mode.

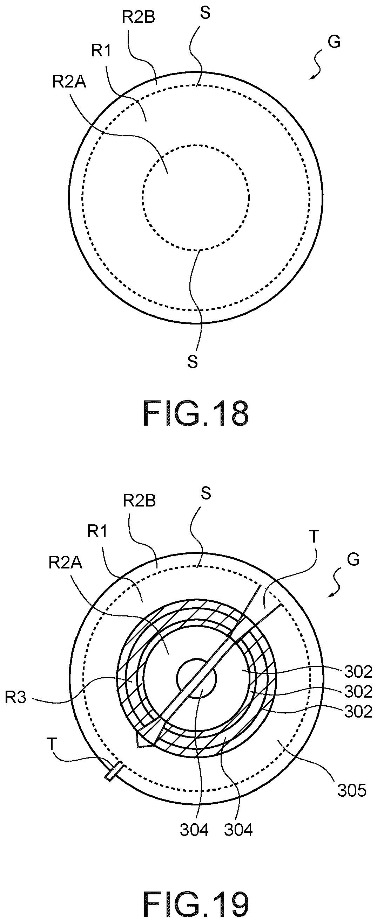

The area detection unit 143 detects each area in the captured image. Fig. 18 is a schematic diagram showing the inverted area R1, the center area R2A, and the outer peripheral area R2B in the captured image G captured in the case where the reflection front lens 101B is attached to an eye.

First, the area detection unit 143 detects the inverted area R1 and the center area R2A. The area detection unit 143 is capable of detecting the inverted area R1 by using object recognition, edge detection, difference between captured images, or the like similarly to the case of the direct-view front lens 101A. Further, the area detection unit 143 is capable of detecting the area inside the detected inverted area R1 as the center area R2A.

Further, the area detection unit 143 detects an unnecessary area. Fig. 19 is a schematic diagram showing an unnecessary area R3 (shaded portion in the figure). As shown in the figure, the unnecessary area R3 is an area appeared also in the center area R2A, of the inverted area R1, and is an area corresponding to the iris 302 and the pupil 304 in the inverted area R1.

The area detection unit 143 is also capable of detecting the unnecessary area R3 by using the difference in color or texture between the corner angle 305 and the iris 302. Further, the area detection unit 143 is also capable of detecting the unnecessary area R3 by estimating the range to be the unnecessary area R3 on the basis of the inclined angle of the mirror 123 obtained in advance.

Further, the area detection unit 143 is capable of causing, in the case where the corner angle 305 is moved to the periphery of the center area R2A, the unnecessary area R3 to include also the area of the corner angle 305. The area detection unit 143 supplies the detected range of each of the inverted area R1, the center area R2A, and the unnecessary area R3 to the image generation unit 144.

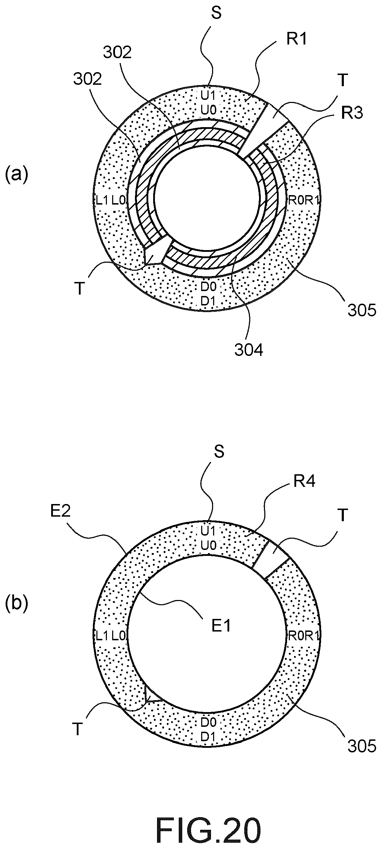

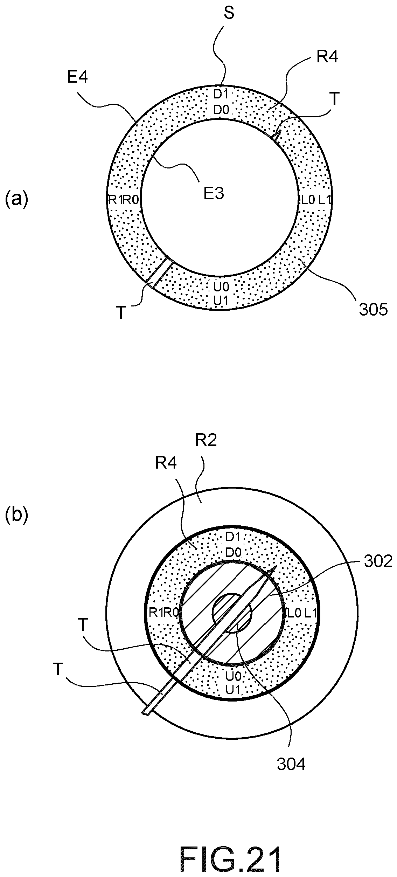

The image generation unit 144 performs correction processing on the captured image to generate a display image. Fig. 20 and Fig. 21 are each a schematic diagram showing an aspect of the correction processing by the image generation unit 144. First, the image generation unit 144 extracts the inverted area R1 in the captured image G as shown in Part (a) of Fig. 20. In the figure, R0, R1, L0, L1, U0, U1, D0, and D1 are each a symbol indicating the position in the inverted area R1.

Subsequently, the image generation unit 144 deletes the unnecessary area R3 in the inverted area R1 as shown in Part (b) of Fig. 20. Hereinafter, the inverted area R1 from which the unnecessary area R3 has been deleted will be referred to as an inverted area R4. In the figure, the inner periphery of the inverted area R4 is shown as an inner periphery E1, and the outer periphery of the inverted area R4 is shown as an outer periphery E2.

Further, the image generation unit 144 inverts the inverted area R4 so as to be point-symmetric with respect to the center of the inverted area R4 as shown in Part (a) of Fig. 21. In the figure, an inner periphery E3 of the inverted area R4 corresponds to the outer periphery E2 before the inversion, and an outer periphery E4 of the inverted area R4 corresponds to the inner periphery E1 before the inversion. That is, the image generation unit 144 performs keystone correction and inverts the inverted area R4 so that the length of the inner periphery E1 matches with the outer periphery E4 and the length of the outer periphery E2 matches with the inner periphery E3. Further, in the case where there is a part of the unnecessary area R3 in the center area R2A, the image generation unit 144 deletes this part.

Further, the image generation unit 144 synthesizes the inverted area R4, which has been inverted, with the non-inverted area R2 (the center area R2A and the outer peripheral area R2B) as shown in Part (b) of Fig. 21. At this time, the image generation unit 144 expands the inverted area R4 or the center area R2A so that the inner periphery E3 of the inverted area R4 matches with the outer periphery of the center area R2A. Further, the image generation unit 144 causes the outer periphery E4 of the inverted area R4 to matches with the inner periphery of the outer periphery area.

In this way, the image generation unit 144 is capable of generating a display image in which the inversion has been eliminated as shown in Fig. 14. The image generation unit 144 supplies the generated display image to the image output unit 145.

The image output unit 145 outputs the display image to the display apparatus 105, and causes the display apparatus 105 to display the display image. The image output unit 145 is capable of rotating the display image in accordance with the positional relationship between a surgeon and an assistant and causing a display to display it, as described above.

### [Operation of Image Processing Apparatus]

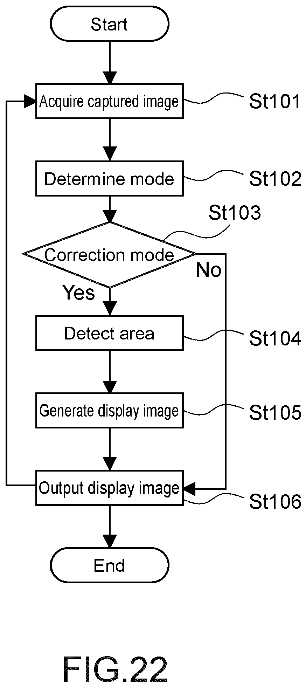

Fig. 22 is a flowchart showing the operation of the image processing apparatus 104. As shown in the figure, first, the image acquisition unit 141 acquires a captured image (St101).

Subsequently, the mode determination unit 142 determines any of the correction mode and the normal mode (St102). The mode determination unit 142 is capable of selecting the correction mode in the case of detecting the front lens 101 in the captured image or receiving a user instruction, and the normal mode in other cases, as described above.

In the case where the mode determination unit 142 has selected the correction mode (St103: Yes), the area detection unit 143 detects the inverted area R1 (St104). Further, in the case where the reflection front lens 101B is attached to the eye, the area detection unit 143 detects the center area R2A and the unnecessary area R3 in addition to the inverted area R1.

Subsequently, the image generation unit 144 performs correction and synthesis processing to generate a display image (St105). The image generation unit 144 inverts the inverted area R1 in the case of the direct-view front lens 101A, and synthesizes it with the non-inverted area R2. Further, in the case of the reflection front lens 101B, the image generation unit 144 inverts the inverted area R4 after deleting the unnecessary area R3, and synthesizes it with the non-inverted area R2.

Further, in the case where the mode determination unit 142 has selected the normal mode (St103: No), the image generation unit 144 uses the captured image as a display image as it is.

Subsequently, the image output unit 145 outputs the display image generated by the image generation unit 144 to the display apparatus 105 (St106), and causes the display apparatus 105 to display the display image. The image output unit 145 is capable of rotating the display image in accordance with the positional relationship between the surgeon and the assistant.

The image processing apparatus 104 performs the above operation. Since the image processing apparatus 104 eliminates the inversion of an image by the front lens 101, a user is capable of performing an operation without being confused.

### [Operation of Ophthalmic Observation System]

The image processing apparatus 104 causes the display apparatus 105 to display the display image in which the inversion of an image by the front lens 101 has been eliminated. An operator performs treatment while viewing the display image. In the case of performing fine work, he/she desires to magnification observation in some cases. In the case where it is sufficient with digital zoom, he/she handles it with digital zoom. In the case where he/she desires to further magnify it, it is necessary to optically magnify it after moving the microscope 102 to an appropriate position in parallel with the X-Y direction.

Since the display image is a corrected image, in the case where he/she desires to view the magnified upper left on the display apparatus 105, for example, it is necessary to move the microscope 102 itself to the lower right, resulting in inconsistency. In this regard, in the case where the operation is performed in the correction mode, the microscope control unit 107 is capable of preventing an operator from being confused by inverting the X-Y operation signal obtained from the microscope control input unit 106 or the like and reflecting it in the movement of the microscope 102.

### [Effect by Ophthalmic Observation System]

From the past, it has been common to observe a part that is difficult to visually recognize from the front of the eye, such as a corner angle, by a front lens and a microscope. In the ophthalmic observation system 100, by performing image processing by the image processing apparatus 104 in addition to the front lens 101 and the microscope 102, it is possible to eliminate the inversion of an image, which causes an operator to be confused.

Further, since the optical system (the front lens 101 and the microscope 102) of the ophthalmic observation system 100 is a simple optical system, it is possible to reduce the attenuation of light and deal with a low amount of light. Further, by inverting the operation signal of the microscope 102 in accordance with the elimination of the inversion of an image, an operator is capable of moving the field of view the microscope 102 without feeling uncomfortable.

### [Hardware Configuration]

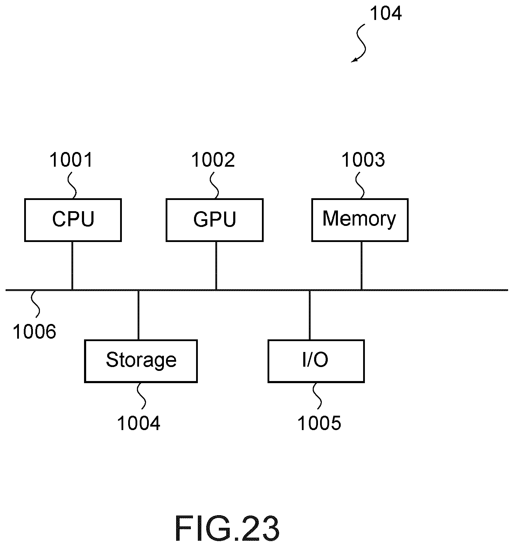

Fig. 23 is a schematic diagram showing a hardware configuration of the image processing apparatus 104. As shown in the figure, the image processing apparatus 104 includes a CPU 1001, a GPU 1002, memory 1003, a storage 1004, and an input/output unit (I/O) 1005 as the hardware configuration. They are connected to each other via a bus 1006.

The CPU (Central Processing Unit) 1001 controls other configurations in accordance with programs stored in the memory 003, processes data in accordance with the program, and stores a result of the process in the memory 1003. The CPU 1001 may be a microprocessor.

The GPU (Graphic Processing Unit) 1002 executes image processing under the control of the CPU 1001. The GPU 1002 may be a microprocessor.

The memory 1003 stores programs to be executed by the CPU 1001 and data. The memory 1003 may be a RAM (Random Access Memory).

The storage 1004 stores programs and data. The storage 1004 may be an HDD (hard disk drive) or an SSD (solid state drive).

The input/output unit 1005 receives inputs to the image processing apparatus 104, and supplies outputs from the image processing apparatus 104 to the outside. The input/output unit 1005 includes an input device such as a keyboard and a mouse, an output device such as a display, and a connection interface such as a network.

The hardware configuration of the image processing apparatus 104 is not limited thereto as long as it is possible to achieve the functional configurations of the image processing apparatus 104. In addition, the entire hardware configuration described above or a part of the hardware configuration may be present on a network.

### (Second Embodiment)

An ophthalmic observation system according to a second embodiment of the present technology will be described.

### [Configuration of Ophthalmic Observation System]

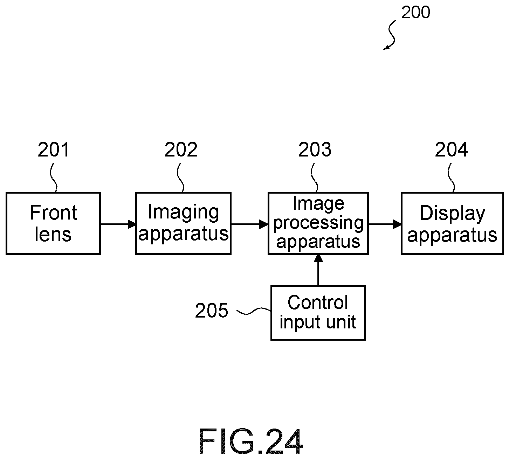

Fig. 24 is a block diagram showing a configuration of an ophthalmic observation system 200 according to this embodiment. As shown in the figure, the ophthalmic observation system 200 includes a front lens 201, an imaging apparatus 202, an image processing apparatus 203, a display apparatus 204, and a control input unit 205. Note that regarding the structure of an eye, the same reference symbols as those in the first embodiment (Fig. 2 and Fig. 3) will be used.

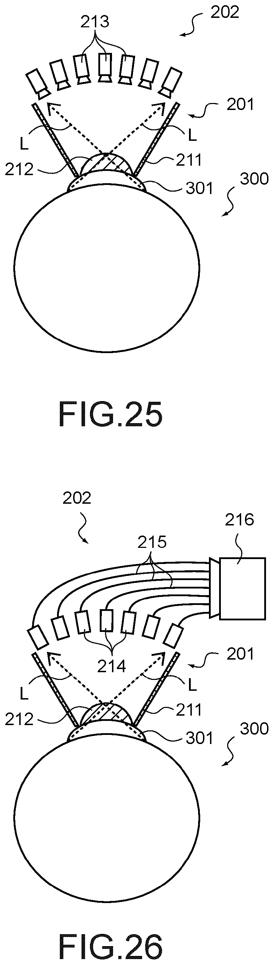

The front lens 201 is a lens to be attached to an eye. In this embodiment, the imaging apparatus 202 is incorporated in the front lens 201. Fig. 25 to Fig. 28 are each a schematic diagram showing a configuration of the front lens 201 and the imaging apparatus 202.

The front lens 201 can be a reflection front lens or a direct-view front lens similarly to the first embodiment. In this embodiment, as the front lens 201, a configuration of a part of the front lens is shown. As shown in Fig. 25 to Fig. 28, the front lens 201 is attached to the eye 300, and includes a support member 211 and a convex lens 212.

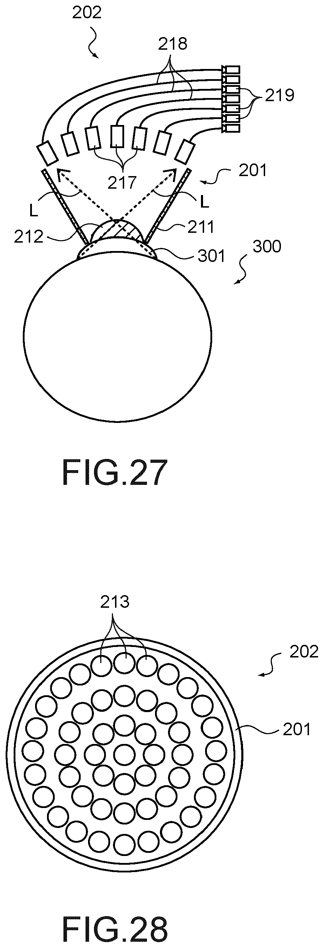

As shown in Fig. 25, the imaging apparatus 202 can have a camera array in which a plurality of small cameras 213 is arranged. Fig. 28 is a schematic diagram showing the arrangement of the small cameras 213, and is a diagram as viewed from the front direction of the eye. As shown in the figure, the small cameras 213 can be concentrically arranged. Further, the arrangement of the small cameras 213 is not limited to such arrangement as long as the entire circumference of the convex lens 212 can be imaged by the array of the small cameras 213.

As shown in Fig. 25, light L emitted from the eye passes through the convex lens 212, enters the corresponding small camera 213, and is imaged.

Further, as shown in Fig. 26, the imaging apparatus 202 may include a plurality of light collection units 214, optical fibers 215 connected to the respective light collection units 214, and one camera 216 to which each of the optical fibers 215 is connected. The light collection units 214 each include an arbitrary optical system such as a lens, and collect incident light on the respective optical fibers 215. The arrangement of the light collection units 214 can be similar to the arrangement of the small cameras 213 shown in Fig. 28, but can be any arrangement as long as the entire circumference of the convex lens 212 can be imaged.

As shown in Fig. 26, the light L emitted from the eye passes through the convex lens 212, and enters the corresponding light collection unit 214. The light that has entered the corresponding light collection unit 214 is guided to the camera 216 by the corresponding optical fiber 215, and imaged by the camera 216.

Further, as shown in Fig. 27, the imaging apparatus 202 may include a plurality of light collection units 217, optical fibers 218 connected to the respective light collection units 217, and a plurality of small cameras 219 to which each of the optical fibers 218 is connected. The light collection units 217 each include an optical system such as a lens, and collect incident light on the respective optical fibers 218. The arrangement of the light collection units 217 can be similar to the arrangement of the small cameras 213 shown in Fig. 28, but can be any arrangement as long as the entire circumference of the convex lens 212 can be imaged.

As shown in Fig. 27, the light L emitted from the eye passes through the convex lens 212, and enters the corresponding light collection unit 217. The light that has entered the corresponding light collection unit 217 is guided to the corresponding small camera 219 by the corresponding optical fiber 218, and imaged by the corresponding small camera 219.

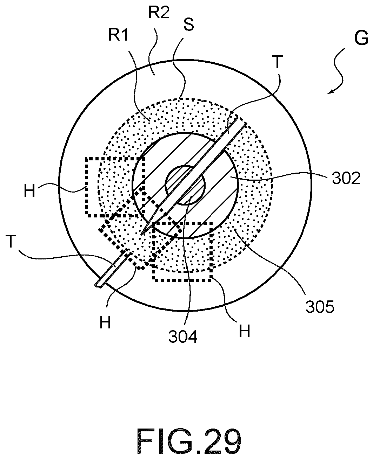

Fig. 29 is a schematic diagram showing an image of the eye, which is imaged by the small cameras 213. In the figure, the imaging range of each of the small cameras 213 is shown as an imaging range H. As shown in the figure, the image of the entire eye can be obtained by combining the imaging ranges of the small cameras 213 with each other.

As shown in Fig. 29, an image of the corner angle 305 shown by the front lens 201 appears around the iris 302, the image is inverted in the area inside the periphery S of the front lens 201. Further, the image is magnified to a certain extent by the front lens 201. In Fig. 29, the area in which the image is inverted is shown as the inverted area R1, and the area in which the image is not inverted is shown as the non-inverted area R2.

Note that also an image of the eye, which is imaged by the small cameras 219 (see Fig. 27), is similar to that imaged by the small cameras 213. Further, in the case of the camera 216 (see Fig. 26), one image as shown in Fig. 29 is captured, and light that enters the corresponding light collection units 214 is light in the range corresponding to the corresponding imaging range H.

The front lens 201 and the imaging apparatus 202 have the configuration as described above. The front lens 201 only needs to be one that is attached to the eye and inverts a part of the image. Typically, the front lens 201 is a gonio lens for observing the corner angle 305. Further, the front lens 201 may be a wide-view lens to be used for a wide-view system for observing a retina.

The image processing apparatus 203 performs imaging processing on the captured image output from the imaging apparatus 202, and generates a display image. The image processing apparatus 203 inverts the inverted area R1 in the captured image to correct it in the correct orientation. Details of this will be described below. The image processing apparatus 203 outputs the generated display image to the display apparatus 204 for display.

The display apparatus 204 displays the display image output from the image processing apparatus 203. The display apparatus 204 is a general display or head mounted display. Further, the display apparatus 204 may include a plurality of displays, e.g., a display for a surgeon and a display for an assistant.

The control input unit 205 accepts an operation input to the image processing apparatus 203 by a user. The user is capable of performing an operation input such as designation of a display mode by using the control input unit 205. The control input unit 205 is, for example, a foot switch.

### [Functional Configuration of Image Processing Apparatus]

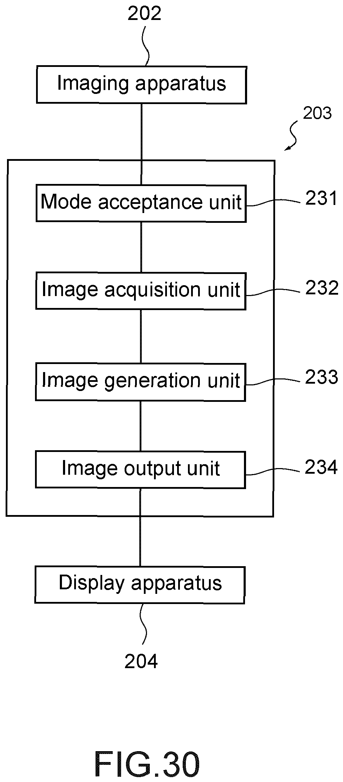

Fig. 30 is a block diagram showing a functional configuration of the image processing apparatus 203. As shown in the figure, the image processing apparatus 203 includes a mode acceptance unit 231, an image acquisition unit 232, an image generation unit 233, and an image output unit 234.

The mode acceptance unit 231 accepts an input of a display mode by a user. The display mode include information regarding magnification, stereoscopic effect, and position in the X-Y direction (hereinafter, referred to as X-Y position information). In the case of accepting the display mode, the mode acceptance unit 231 instructs the image acquisition unit 232 of the display mode.

The image acquisition unit 232 acquires the captured image as shown in Fig. 29 from the imaging apparatus 202. The image acquisition unit 232 supplies the acquired captured image to the image generation unit 233.

The image generation unit 233 performs correction processing on the captured image, and generates a display image. The image generation unit 233 calculates the surgical field range determined in accordance with the magnification and the X-Y position information specified in the display mode, and extracts a captured image of the imaging range H in which the surgical field range appears.

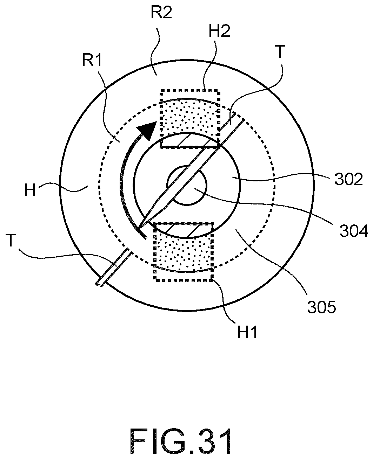

The image generation unit 233 changes the position and orientation of the extracted captured image and performs panorama synthesis. Fig. 31 is a schematic diagram showing the change in position and orientation of the captured image by the image generation unit 233, and the captured image of an imaging range H1 is inverted to a range H1. As shown in the figure, the image generation unit 233 changes the position and orientation of the captured image so that the inversion of the inverted area R1 is eliminated.

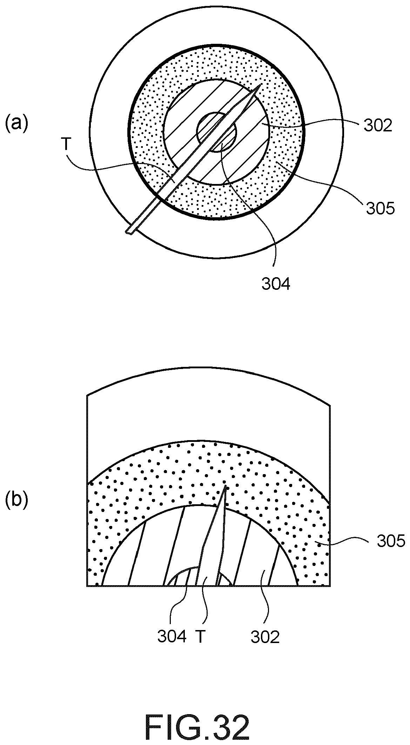

Fig. 32 is a schematic diagram showing a display image generated by the image generation unit 233. In the case where the image of the entire eye is instructed by the display mode, the image generation unit 233 generates a display image using the captured images of all the imaging ranges H as shown in Part (a) of Fig. 32.

Further, in the case where the image in which a part of the eye is magnified is instructed by the display mode, the image generation unit 233 generates a display image by using a captured image of a part of the imaging range H as shown in Part (b) of Fig. 32.

The image generation unit 233 is capable of generating a display image in which the inversion of the inverted area R1 has been eliminated in this way. Further, the image generation unit 233 is also capable of changing the parallax by selecting the imaging range H so as to change the convergence, i.e., by generating a display image from the imaging ranges H away from each other. The image generation unit 233 supplies the generated display image to the image output unit 234.

The image output unit 234 outputs the display image to the display apparatus 204, and causes the display apparatus 204 to display the display image. Similarly to the first embodiment, the image output unit 234 is capable of causing a display to display the display image viewed from the position of the surgeon and another display to display the display image rotated in accordance with the position of the assistant.

### [Operation of Image Processing Apparatus]

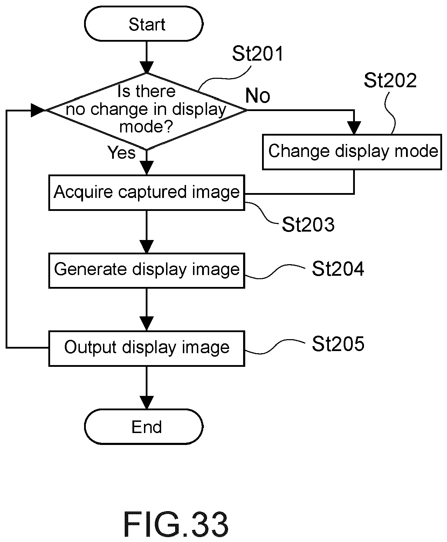

Fig. 33 is a flowchart showing the operation of the image processing apparatus 203. As shown in the figure, first, the mode acceptance unit 231 accepts an input of the display mode (St201). In the case where there is a change in display mode (St201: No), the display mode is changed (St202). After the display mode is changed or there is no change in display mode (St201: Yes), the image acquisition unit 232 acquires a captured image in accordance with the display mode (St203).

Subsequently, the image generation unit 233 changes the orientation and position of the captured image for each imaging range H in accordance with the display mode and synthesizes them to generate a display image (St205). The image output unit 234 outputs the display image to the display apparatus 204 for display (St206).

Since the image processing apparatus 203 does not use optical zoom for imaging a captured image, it is possible to simultaneously present a magnified display image and a display image of the entire eye, and prevent a surgeon from losing the position of the magnified part.

Further, the surgical field needs to be magnified in the case where a fine procedure is performed. In such a case, not only a magnified image but also a stereoscopic effect due to parallax is important in many cases. In the present technology, since the parallax can be easily changed by selecting two imaging ranges so as to change the convergence, it is possible to instantly respond to setting parameters for the stereoscopic effect specified by a user.

Further, in this embodiment, since an image of the surgical field is provided by image processing without using optical zoom, it is also possible to use the image in such a way that a surgeon views a magnified image and an assistant views the whole image.

### [Effect by Ophthalmic Observation System]

In the ophthalmic observation system 200, by performing image processing by the image processing apparatus 203 in addition to the front lens 201, it is possible to eliminate the inversion of an image that causes an operator to be confused.

Further, since the optical system (front lens 201) of the ophthalmic observation system 200 is a simple optical system, it is possible to reduce the attenuation of light and deal with a low amount of light.

Further, in the ophthalmic observation system 200, it is possible to simultaneously present a whole image of an eye and a magnified view of the eye, and instantly change the surgical field. Further, in the ophthalmic observation system 200, the parallax can be easily changed, and it is possible to control the stereoscopic effect and present appropriate display images for a surgeon and assistant.

### [Hardware Configuration]

The image processing apparatus 203 can be realized by a hardware configuration similar to that of the image processing apparatus 104 according to the first embodiment.

It should be noted that the present technology may take the following configurations.
(1) An image processing apparatus, including:
   an image generation unit that inverts an inverted area in a captured image captured via a front lens attached to an eye, the inverted area being an image area in which an image is inverted by the front lens, and generates a display image.
(2) The image processing apparatus according to (1) above, further including
   an area detection unit that detects the inverted area in the captured image.
(3) The image processing apparatus according to (1) or (2) above, in which
   the image generation unit inverts the inverted area with a center of the inverted area as a center point.
(4) The image processing apparatus according to any one of (1) to (3) above, in which
   the image generation unit inverts the inverted area and synthesizes the area with a non-inverted area to generate the display image, the non-inverted area being an image area in which an image is not inverted by the front lens in the captured image.
(5) The image processing apparatus according to (4) above, in which
   the front lens is a direct-view front lens that includes a concave lens and a convex lens, the concave lens being in contact with a cornea, the convex lens refracting light emitted from the concave lens toward a front direction of the eye, and
   the inverted area is circular and the non-inverted area surrounds a periphery of the inverted area.
(6) The image processing apparatus according to (5) above, in which
   the image generation unit inverts the inverted area, and synthesizes the inverted area with the non-inverted area by causing an outer periphery of the inverted area, which has been inverted, and an inner periphery of the non-inverted area to match with each other.
(7) The image processing apparatus according to (4) above, in which
   the front lens is a reflection front lens that includes a convex lens and a mirror, the convex lens being in contact with a cornea, the mirror being circumferentially disposed around the convex lens and reflecting light emitted from the convex lens toward a front direction of the eye, and
   the inverted area is a circumferential area and the non-inverted area includes a center area and an outer peripheral area, the center area being surrounded by the inverted area, the outer peripheral area surrounding the inverted area.
(8) The image processing apparatus according to (7) above, in which
   the image generation unit detects, as an unnecessary area, an area including an image of the center area in the inverted area, deletes, the unnecessary area, inverts the inverted area, and synthesizes the inverted area, the center area, and the outer peripheral area with each other by causing an inner periphery of the inverted area, which has been inverted, and an outer periphery of the center area to match with each other and causing an outer periphery of the inverted area, which has been inverted, and an inner periphery of the center area to match with each other.
(9) The image processing apparatus according to any one of (2) to (8) above, further including
   a mode determination unit that detects that the captured image incudes the front lens, selects a correction mode for correcting inversion of an image by the front lens, and notifies the area detection unit and the image generation unit of the correction mode, in which
   the area detection unit detects, where the mode determination unit has selected the correction mode, the inverted area.
(10) The image processing apparatus according to (9) above, in which
   the mode determination unit detects the front lens by object recognition processing on the captured image.
(11) The image processing apparatus according to (9) above, in which
   the mode determination unit detects the front lens by detecting a marker attached to the front lens in the captured image.
(12) The image processing apparatus according to any one of (2) to (11) above, in which
   the area detection unit detects the inverted area by object recognition processing on the captured image.
(13) The image processing apparatus according to any one of (2) to (12) above, in which
   the area detection unit detects the inverted area by using a difference in texture due to a structure of the eye in the captured image.
(14) The image processing apparatus according to any one of (2) to (12) above, in which
   the area detection unit detects the inverted area by edge detection processing on the captured image.
(15) The image processing apparatus according to any one of (2) to (12) above, in which
   the area detection unit detects the inverted area on a basis of a difference between the captured image and a captured image of the eye to which the front lens is not attached.
(16) The image processing apparatus according to any one of (2) to (12) above, in which
   the area detection unit detects the inverted area by using depth information extracted from parallax information obtained from the captured image.
(17) The image processing apparatus according to any one of (1) to (16) above, in which
   the captured image includes a plurality of images captured for each predetermined imaging range, and
   the image generation unit inverts a position and an orientation of each of the plurality of images to generate the display image.
(18) An ophthalmic observation apparatus, including:
   a front lens that is attached to an eye and inverts an image; and
   an image processing apparatus including an image generation unit that inverts an inverted area in a captured image captured via the front lens, the inverted area being an image area in which an image is inverted by the front lens, and generates a display image.
(19) The ophthalmic observation apparatus according to (18) above, in which
   the front lens is a gonio lens.
(20) An ophthalmic observation system, including:
   a front lens that is attached to an eye and inverts an image;
   a microscope that magnifies emitted light of the front lens;
   a microscope control input unit that accepts an operation input by a user and generates an input signal;
   an imaging apparatus that is connected to the microscope and captures an image via the front lens and the microscope;
   an image processing apparatus including an image generation unit that inverts an inverted area in the captured image captured via the front lens, the inverted area being an image area in which an image is inverted by the front lens, and generates a display image; and
   a microscope control unit that inverts, where the image generation unit has inverted the inverted area, the input signal to control the microscope.

### Reference Signs List

- 100, 200: ophthalmic observation system
- 101, 201: front lens
- 102: microscope
- 103, 202: imaging apparatus
- 104, 203: image processing apparatus
- 105, 204: display apparatus
- 106: microscope control input unit
- 107: microscope control unit
- 108: user input unit
- 141, 232: image acquisition unit
- 142: mode determination unit
- 143: area detection unit
- 144, 233: image generation unit
- 145, 234: image output unit
- 205: control input unit
- 231: mode acceptance unit

## Claims

1. An image processing apparatus, comprising:
an image generation unit that inverts an inverted area in a captured image captured via a front lens attached to an eye, the inverted area being an image area in which an image is inverted by the front lens, and generates a display image.

2. The image processing apparatus according to claim 1, further comprising an area detection unit that detects the inverted area in the captured image.

3. The image processing apparatus according to claim 1, wherein
the image generation unit inverts the inverted area with a center of the inverted area as a center point.

4. The image processing apparatus according to claim 3, wherein
the image generation unit inverts the inverted area and synthesizes the area with a non-inverted area to generate the display image, the non-inverted area being an image area in which an image is not inverted by the front lens in the captured image.

5. The image processing apparatus according to claim 4, wherein
the front lens is a direct-view front lens that includes a concave lens and a convex lens, the concave lens being in contact with a cornea, the convex lens refracting light emitted from the concave lens toward a front direction of the eye, and
the inverted area is circular and the non-inverted area surrounds a periphery of the inverted area.

6. The image processing apparatus according to claim 5, wherein
the image generation unit inverts the inverted area, and synthesizes the inverted area with the non-inverted area by causing an outer periphery of the inverted area, which has been inverted, and an inner periphery of the non-inverted area to match with each other.

7. The image processing apparatus according to claim 4, wherein
the front lens is a reflection front lens that includes a convex lens and a mirror, the convex lens being in contact with a cornea, the mirror being circumferentially disposed around the convex lens and reflecting light emitted from the convex lens toward a front direction of the eye, and
the inverted area is a circumferential area and the non-inverted area includes a center area and an outer peripheral area, the center area being surrounded by the inverted area, the outer peripheral area surrounding the inverted area.

8. The image processing apparatus according to claim 7, wherein
the image generation unit detects, as an unnecessary area, an area including an image of the center area in the inverted area, deletes, the unnecessary area, inverts the inverted area, and synthesizes the inverted area, the center area, and the outer peripheral area with each other by causing an inner periphery of the inverted area, which has been inverted, and an outer periphery of the center area to match with each other and causing an outer periphery of the inverted area, which has been inverted, and an inner periphery of the center area to match with each other.

9. The image processing apparatus according to claim 2, further comprising
a mode determination unit that detects that the captured image incudes the front lens, selects a correction mode for correcting inversion of an image by the front lens, and notifies the area detection unit and the image generation unit of the correction mode, wherein
the area detection unit detects, where the mode determination unit has selected the correction mode, the inverted area.

10. The image processing apparatus according to claim 9, wherein
the mode determination unit detects the front lens by object recognition processing on the captured image.

11. The image processing apparatus according to claim 9, wherein
the mode determination unit detects the front lens by detecting a marker attached to the front lens in the captured image.

12. The image processing apparatus according to claim 2, wherein
the area detection unit detects the inverted area by object recognition processing on the captured image.

13. The image processing apparatus according to claim 2, wherein
the area detection unit detects the inverted area by using a difference in texture due to a structure of the eye in the captured image.

14. The image processing apparatus according to claim 2, wherein
the area detection unit detects the inverted area by edge detection processing on the captured image.

15. The image processing apparatus according to claim 2, wherein
the area detection unit detects the inverted area on a basis of a difference between the captured image and a captured image of the eye to which the front lens is not attached.

16. The image processing apparatus according to claim 2, wherein
the area detection unit detects the inverted area by using depth information extracted from parallax information obtained from the captured image.

17. The image processing apparatus according to claim 1, wherein
the captured image includes a plurality of images captured for each predetermined imaging range, and
the image generation unit inverts a position and an orientation of each of the plurality of images to generate the display image.

18. An ophthalmic observation apparatus, comprising:
a front lens that is attached to an eye and inverts an image; and
an image processing apparatus including an image generation unit that inverts an inverted area in a captured image captured via the front lens, the inverted area being an image area in which an image is inverted by the front lens, and generates a display image.

19. The ophthalmic observation apparatus according to claim 18, wherein
the front lens is a gonio lens.

20. An ophthalmic observation system, comprising:
a front lens that is attached to an eye and inverts an image;
a microscope that magnifies emitted light of the front lens;
a microscope control input unit that accepts an operation input by a user and generates an input signal;
an imaging apparatus that is connected to the microscope and captures an image via the front lens and the microscope;
an image processing apparatus including an image generation unit that inverts an inverted area in the captured image captured via the front lens, the inverted area being an image area in which an image is inverted by the front lens, and generates a display image; and
a microscope control unit that inverts, where the image generation unit has inverted the inverted area, the input signal to control the microscope.
